# EUROPEAN PATENT SPECIFICATION

(11) **EP 1 582 235 B1**
(45) Date of publication and mention of the grant of the patent: **25.05.2011**
(21) Application number: 05003645.8
(22) Date of filing: 21.02.2005
(51) Int. Cl.: A61N 1/372, A61N 1/375

(54) **Implantable medical device with slot antenna**
Implantierbare medizinische Vorrichtung mit Schlitzantenne
Dispositif médical implantable avec antenne à fente

(30) Priority: 30.03.2004 SE 0400849
(43) Date of publication of application: 05.10.2005
(73) Proprietor: St. Jude Medical AB, 175 84 Järfälla (SE)
(72) Inventor: Edvardsson, Kurt Olov, 582 45 Linköping (SE)

(56) References cited:
- EP-A- 1 493 465
- US-A1- 2002 095 195

## Description

### FIELD OF INVENTION

The present invention relates to an implantable medical device.

### DESCRIPTION OF RELATED ART AND BACKGROUND OF THE INVENTION

In an implantable medical device, such as a cardiac pacemaker, an implantable cardioverter/defibrillator, or an insulin dispenser, telemetry is used to e.g. change or modify operation characteristics of the implantable device or to readout data from the implantable medical device to monitor its function or to achieve information of the patient having the device implanted. Telemetry systems for implantable medical devices utilize radio-frequency energy to enable communication between the implantable device and an external programmer device.

Earlier telemetry systems used a rather low radio frequency, i.e. 8-300 kHz, as a carrier wavelength for communication between an antenna of the implantable device and an antenna of the external programmer device, which were inductively coupled to each other. Due to the very poor operating distance of this technique, the exterior antenna had to be located in close proximity to the implantable device, typically within a few inches. Further, the communication suffered from low transmission data rate.

Recently, telemetry systems using a radio frequency data link operating at a much higher frequency, around 400 MHz, have been proposed, which enable two improvements to be made. Firstly, the antenna efficiency can be improved allowing extended range between the pacemaker and the external antenna. Secondly, the transmission data rate can be improved. Even higher frequencies can be used such as those within the ISM-band at 2400-2485.5 MHz.

EP 1 493 465 A1 discloses an implantable medical device with a conductive housing enclosing electric circuitry and radio frequency circuitry. At least one slot is provided in the conductive housing with a slot feeding operatively interconnected between the radio frequency circuitry and the slot.

US 2002/0095195 A1 discloses an implantable medical device utilizing such far-field electromagnetic radiation to allow communication over a large distance. Two conductive halves of a housing for the implantable device act as a dipole antenna for radiating and receiving far-field radio frequency radiation modulated with telemetry data. An insulating header, in which therapy leads can be located, separates the conductive halves.

### SUMMARY OF THE INVENTION

US 2002/0095195 A1 discloses a manner to utilize the limited space for the antenna function, but, nevertheless, there are several limitations of using an implantable medical device, in which two separated conductive halves of the housing act as a dipole antenna.

Firstly, there is arranged a header made of dielectric material between the two conductive halves, thereby admitting external interfering radiation to enter the implantable device and interfere with signals transmitted within any of the two conductive halves or with signals transmitted across the dielectric header.

Secondly, in order to hermetically seal the two housing halves, a number of feed-throughs between them are needed since different electric circuitry is located in different housing halves to effectively use the available space.

Thirdly, the design of the implantable medical device does not allow for an optimum location of the therapy leads with respect to potential interference of the therapy signals by radio frequency signals fed to or received by the dipole antenna. The antenna type lacks a voltage node on its surface, where the electric field has a minimum, thereby affecting the therapy signals to a minimum extent.

Finally, the mechanical structure of the prior art implantable medical device seems not to be optimum: two housing portions of a conductive material have to produced, and to be fixed to and hermetically sealed against an intermediate piece of dielectric material. The manufacturing is made further complicated by the need of a number of feed-throughs.

It is thus an object of the present invention to provide an implantable medical device provided with an antenna that overcomes the above-mentioned problems.

In this respect there is a particular object of the invention to provide such an implantable medical device, which exhibits an overall improved antenna performance in comparison with implantable medical devices of the prior art. Higher antenna efficiency implies increased usable range for an exterior antenna.

A further object of the invention is to provide such an implantable medical device, which is shielded from external radio frequency radiation in any direction.

A still further object of the invention is to provide such an implantable medical device, which has a minimum number of feed-throughs in the housing thereof.

A yet further object of the invention is to provide such an implantable medical device, which is provided with therapy lines protruding from the implantable medical device, where the therapy lines are located so as to be affected as little as possible by radiation transmitted and/or received by the antenna of the implantable medical device.

A still further object of the invention is to provide such an implantable medical device, in which the antenna is easy to manufacture to a low cost, easy to tune, and which antenna enables an efficient use of available space.

A yet further object of the invention is to provide such an implantable medical device, which is reliable, and particularly mechanically durable.

Implantable medical devices as claimed in the appended patent claims attain these objects, among others.

According to the present invention there is provided an implantable medical device according to claim 1. The surface portion of an electrically conductive material may advantageously be a surface portion such as a portion of a dielectric header, which is metallized. The surface portion may be made of sheet metal.

If the housing is an electrically conductive housing, the entire housing with the slot can be tailored to operate as an antenna for the radio frequency telemetry signals without any requirement of an insulating separation.

Further, the electric circuitry, the radio frequency circuitry, and the interconnection there between are enclosed by the electrically conductive housing to shield the electric circuitry, the radio frequency circuitry, and the interconnection from external radiation in any direction.

If therapy lines, such as those used in a cardiac pacemaker device, are provided, they are advantageously arranged to protrude from the housing close to a voltage node of an electromagnetic field as obtained when the surface portion of the electrically conductive material operates as an antenna for the radio frequency telemetry signals. Such provision provides for a minimum interference between the therapy lines and the antenna function.

Three different general principles regarding the shape and location of the slot is described: a slot which is open in one end thereof and which in the antenna literature often is referred to as a notch antenna; a slot which is closed in both ends thereof and which is preferably provided with a backing cavity; and a slot which is closed in both ends thereof and which is formed as a through-hole to extend across the complete thickness of the implantable medical device.

Several types of slot feeding are known in the antenna literature and some of them are suitable to be used in the present invention. Depending on the kind of slot a conductor crossing the slot, an inductive coupling loop within the slot, a conductor crossing a portion of the slot and connected capacitively to the opposite edge of the slot, or a feeding including a kind of coaxial or wire feed-to-waveguide transition can be used. The latter feeding can preferably be used when the slot is provided with a backing cavity, where the backing cavity operates as a waveguide to feed the slot.

In the description it is to be understood that the antenna of the present invention is operable to transmit or receive radio frequency signals. Even if a term is used herein that suggests one specific signal direction it is to be appreciated that such a situation can cover that signal direction and/or its reverse.

### BRIEF DESCRIPTION OF THE DRAWINGS

Figs. 1a-b illustrate schematically, in a top view with a top cover removed, and in a side view, respectively, an implantable medical device.
Figs. 2a-b illustrate schematically, in a top view with a top cover removed, and in a side view, respectively, a further implantable medical device
Figs. 3a-b illustrate schematically, in a top view with a top cover removed, and in a side view with a slot feeding removed, respectively, a still further implantable medical device.
Fig. 4 illustrates schematically, in a top view with a top cover removed, a yet further implantable medical device.
Fig. 5 illustrates schematically, in a side view with a slot feeding removed, a still further implantable medical device.
Fig. 6 illustrates schematically, in a side view, a slot feeding for use with the implantable medical device of Fig. 5.
Fig. 7 illustrates schematically, in a side view, another slot feeding for use with the implantable medical device of Fig. 5.
Figs. 8a-b illustrate schematically, in a top view with a top cover removed, and in a side view, respectively, a yet further implantable medical device.
Figs. 9-11 illustrate schematically, in top views, still further implantable medical devices.

Throughout the Figures similar parts and components, and portions thereof, are denoted by identical reference numerals.

### DETAILED DESCRIPTION OF PREFERRED EMBODIMENTS

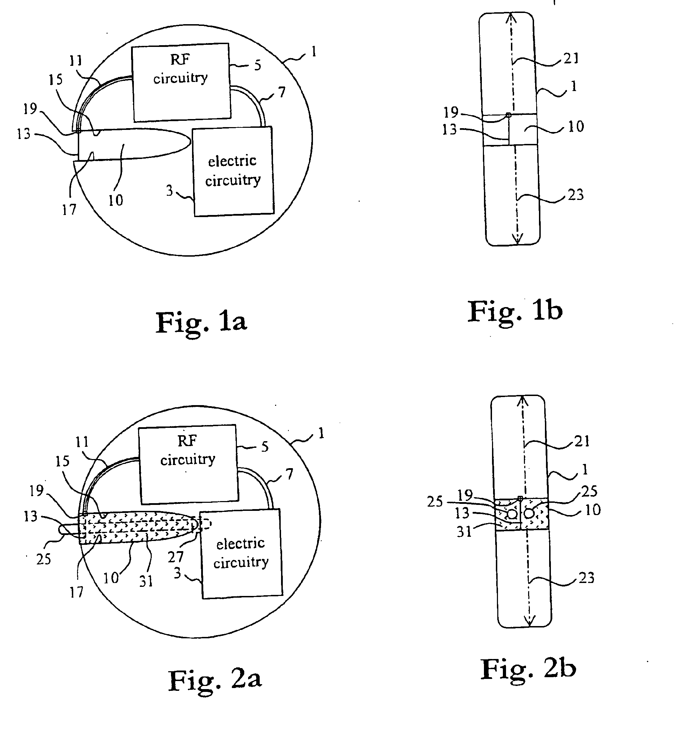

An implantable medical device will be described with reference to Figs. 1a-b. The implantable medical device may e.g. be a pacemaker, an insulin dispenser or other medical equipment including a telemetry link, preferably a high frequency telemetry link.

The implantable medical device comprises an electrically conductive hollow housing 1, electric circuitry 3 for the operation of the implantable medical device, and radio frequency circuitry 5 for transmitting and/or receiving radio telemetry frequency signals. The electric circuitry 3 and the radio frequency circuitry 5 are interconnected 7 and are arranged in the electrically conductive housing in a common space or in separate compartments, which may be shielded from each other.

The electrically conductive housing 1 is provided with a slot 10, and feed conductors 11, 13 are interconnected between the radio frequency circuitry 5 and edges 15, 17 of the slot 10. By means of such provisions, the electrically conductive housing can be adapted to operate as an antenna for the radio frequency telemetry signals.

In the Figs. 1a-b the slot 10 is open in one end thereof to form what is usually referred to as a shunt or notch antenna in the antenna literature, see e.g. R.C. Johnson, Antenna Engineering Handbook, third edition, McGraw-Hill, 1993, pages 37-14 - 37-17. This kind of antenna has been used extensively on aircrafts. The length of the slot should nominally be λ/4 to obtain resonance, where λ is the effective wavelength in the dielectric material in the slot. The length can be different if tuning components are included.

In Figs. 1a-b the slot is open so that blood and surrounding tissue may fill out the slot. The dielectric constant of such matter may vary considerably, which naturally affect the resonance frequency or wavelength of the antenna. The dielectric material is not important for the radiation performance as such, but tunes the resonance to a lower frequency than the one indicated by the physical length. At 400 MHz and a dielectric constant of 1, a quarter of an effective wavelength to obtain resonance measures 18 cm, whereas at a dielectric constant of 65 the resonant slot length is 2.3 cm. Correspondingly, at 2.4 GHz a dielectric constant of 1 gives a resonant λ/4 length of 3.1 cm, whereas a dielectric constant of 65 gives a resonant λ/4 length of 0.39 cm. All these slot lengths but the largest are easily feasible to use in a cardiac pacemaker device, which today typically measures about 3-8 cm in diameter. Also, there is a wide possibility to use shorter slots by suitable impedance matching. The use of a λ/4 slot as being illustrated in Figs. 1a-b is obviously preferred to keep the dimensions small, especially when a frequency around 400 MHz is used.

Preferably, the electric circuitry 3, the radio frequency circuitry 5, and the interconnection 7 are enclosed by the electrically conductive housing 1 so that the electric circuitry 3, the radio frequency circuitry 5, and the interconnection 7 are shielded from external radio frequency radiation in any direction. Hence, the electrically conductive housing 1 operates similarly to a Faraday cage to effectively hinder radio frequency radiation from entering the housing. With respect to the shielding functionality the conductive housing 1 may have openings of a size, which depends on the frequency of the radiation that shall be shielded. As the electrically conductive housing 1 is typically sealed, particularly hermetically sealed, sealed feed-throughs are required.

One of the feed conductors 13 is therefore provided with a hermetic feed-through 19 in the electrically conductive housing 1 so that the feed conductor 13 can protrude from one of the edges 15 of the electrically conductive housing 1, and extend across the slot 10 to be connected at the opposite edge 17 of the slot 10 to create an electric field within the slot 10 by flowing a feeding current in the feed conductor 13.

Preferably, the feed conductors are center 13 and shield 11 conductors of a coaxial cable, where the center conductor 13 is connected at the opposite edge 17 of the slot 10. The conductor may, however, be thicker than an ordinary center conductor of a coaxial cable in the slot 10.

The feeding of the antenna may be implemented in any manner known in the art, e.g. using balanced feed or unbalanced feed, and using any kind of antenna tuning circuit or impedance matching network (not illustrated), which loads the antenna with a variable amount of inductance or capacitance to thereby adjust the effective electrical length of the antenna and match the antenna impedance to the impedance of the transmitter/receiver. In this manner, the reactance of the antenna may be tuned out so that the antenna forms a resonant structure at the specified carrier frequency and efficiently transmits/receives far-field radio frequency radiation. Further, radiation-affecting components may be arranged across the slot 10. Capacitors, inductors, or active components may be interconnected between the edges 15, 17 of the slot 10. Capacitances may be implemented as small protrusions at the edges 15, 17 of the slot 10, whereas inductors may be implemented as narrow strips across slot 10.

The design of the impedance matching network and the slot 10 can be chosen in order to obtain suitable antenna performance in terms of radiation parameters such as resonance frequency, input impedance, bandwidth, radiation pattern, gain, polarization and near-field pattern.

Another way to control the feeding impedance is to move the location of the feed conductor 13 along the slot 10.

Preferably, the radiation parameters can be controlled to accommodate for different filling materials in the slot, which have different dielectric properties.

It shall be appreciated that the feed conductor 13 does not necessarily have to cross the slot in its entire width. The feed conductor 13 may for instance make a loop in the slot and be connected to the same slot edge, at which it is fed through the electrically conductive housing 1.

The slot 10 in the Figs. 1a-b is located substantially halfway between two oppositely located edges of the electrically conductive housing, i.e. so that extensions, of the electrically conductive housing 1 on either side of the slot in directions orthogonal to the slot 10, schematically indicated by arrows 21, 23, are of similar sizes. Such location of the slot provides for capabilities to obtain an optimum antenna performance. A location closer to the circumference of the housing 1 will gradually make the impedance matching more difficult.

Figs. 2a-b illustrate a second implantable medical device wherein the device is provided with two output therapy lines 25 connected to the electric circuitry 3, where each of the therapy lines 25 is provided with a hermetic feed-through 27. The protrusions of the therapy lines 25 are only schematically indicated in Figs. 2a-b and it shall be appreciated by the person skilled in the art that they may be longer. The therapy lines 25 may have different shape and be different in number.

The therapy lines 25 should preferably be located where they have as small influence on the antenna function as possible. This is useful both to avoid influence on the therapy function and to avoid the therapy lines to operate as antennas. In the antenna design procedure an electromagnetic field calculation is typically performed, and one result of such a calculation is the electric field around the implantable medical device, which will give hints for suitable locations. In order to thus minimize the interference the therapy lines 25 are located in the slot 10, preferably symmetrically in the slot, where the electric field has a minimum. An alternative location is at an opposite edge of the electrically conductive housing 1. The shape of the housing 1 may be less regular than what is illustrated in Figs. 2a-b, but still a position on the circumference of the housing 1 can be found, where a local minimum of the electric field exists.

Further, the slot 10 is filled with a dielectric material 31, particularly a plastic, a glass or a ceramic material. This gives on one hand a well-defined dielectric constant of the material 31 in the slot, and on the other hand a good support for the therapy lines 25.

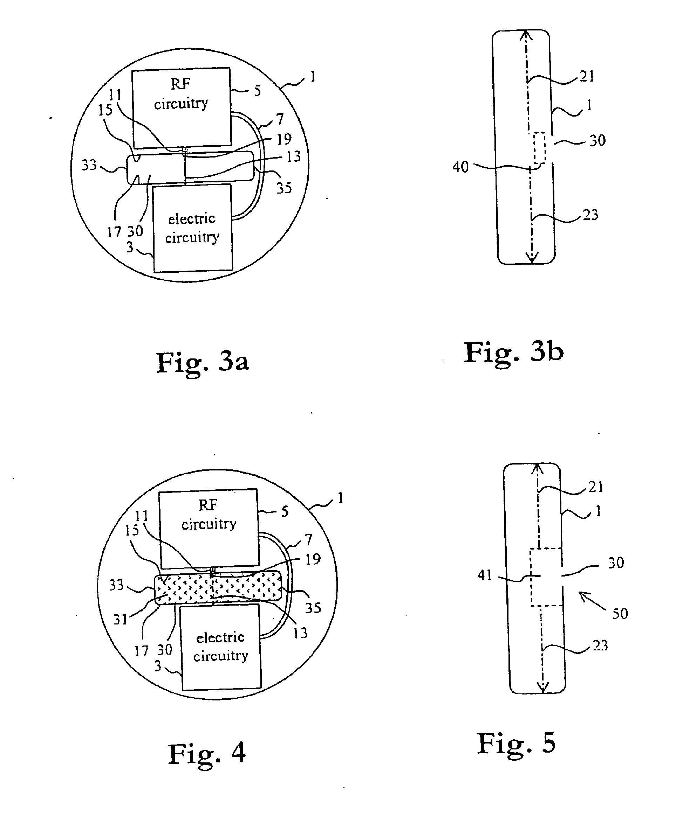

A third implantable medical device will next be described with reference to Figs. 3a-b. The device comprises as above an electrically conductive housing 1, in which electric circuitry 3, and radio frequency circuitry 5 are interconnected. Feed conductors 11, 13 are interconnected between the radio frequency circuitry 5 and edges 15, 17 of the slot, which in this example has different shape, and is therefore denoted by 30. The slot 30 is closed in both ends 33, 35 thereof and the antenna thus formed is commonly referred to as a slot or aperture antenna. A slot antenna is an elongated aperture in a conducting surface where one or more feeding elements generate an electric field over the elongated aperture.

The slot 30 is preferably half an effective wavelength (λ/2) long to achieve a resonant structure, but a shorter slot will radiate as well but with gradually larger impedance matching problems. A very short slot will also have a narrower instantaneous bandwidth. The shape is not critical for the antenna function and for instance rounded and wider ends, a so-called dumbbell shape, is frequently used to decrease the resonant length.

Below the slot 30 there is provided electric shielding or circuitry (schematically indicated at 40 in Fig. 3b) to prevent the slot from radiating inwards in the housing 1 to affect the circuitry therein in an adverse manner. The tissue of the body, which surrounds the implantable medical device, has typically a high electric constant, thereby reducing the length of the λ/2 slot. This also reduces the amount of radiation directed to the interior of the housing 1.

Obviously, there is a natural transition from the λ/2 slot geometry of Figs. 3a-b to the λ/4 notch of Figs. 1a-b. Provided that the λ/4 notch is made in a thin structure it can be seen as a λ/2 slot, which is bent over the edge of the structure.

Fig. 4 illustrates a fourth implantable medical device, which is identical with the Figs. 3a-b embodiment except of that the slot 30 is filled with dielectric material 31 having a known dielectric constant.

Fig. 5 illustrates a fifth implantable medical device wherein the slot is what is referred to as a cavity-backed slot 50, and comprises a backing cavity 41 below the slot 30 instead of the shielding 40. In other respects this device is identical with the device in Figs. 3a-b.

The backing cavity 41 at the inside of the housing 1 is a rather large structure; it may measure λ/2 times λ/4-λ/2, where λ is the effective wavelength in the filling material of the backing cavity 41, if any. The antenna is a cavity resonator fed energized by a feed conductor connected across the slot (not illustrated), which radiates from the slot aperture. Further reference to cavity-backed slot antennas is given in R.C. Johnson, Antenna Engineering Handbook, third edition, McGraw-Hill, 1993, pages 8-7 - 8-9. Depending on the dielectric constant of the filling material the depth of the cavity 41 will be different, but in the case the cavity should be deeper than the thickness of the implantable medical device, the backing cavity can be turned to be e.g. parallel with the surface of the housing 1, in which the thin slot is made.

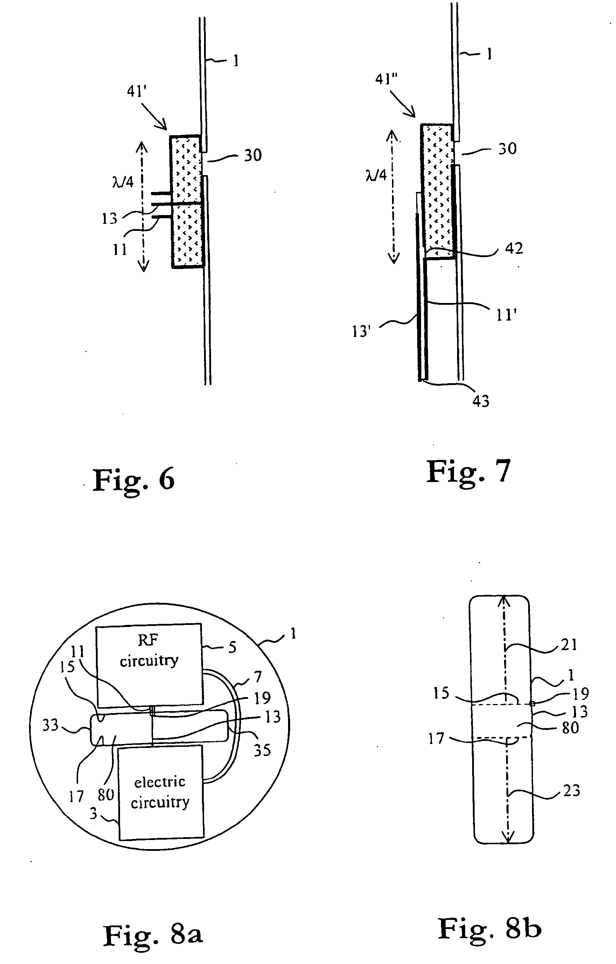

In Fig. 6 such a turned cavity 41' for backing of the slot 30 is illustrated. The cavity can be filled with a ceramic material, which is partly metallized and welded or brazed to the electrically conductive housing to form a hermetic sealing. The feeding of the slot 30 can include a coaxial feed-to-waveguide transition. The center conductor 13 of the coaxial feeding is connected through a hole in the ceramic to an edge of the slot 30, whereas the shield conductor 11 is connected to the metallized ceramic. The ceramic should not be metallized in the slot 30. The length of the cavity 41' is preferably about a quarter of an effective wavelength to obtain high impedance at the slot 30.

In Fig. 7 an alternative feeding of the turned cavity-backed slot 50 is shown. A bottom end of the cavity, here denoted 41", is provided with a second slot 42. A wire 13', possibly on a printed circuit board 43, below the second slot 42 can be adapted to feed the second slot 42 to obtain a wire feed-to-waveguide transition. If the ceramic has a high dielectric constant the second slot 42 will be too short to radiate towards the electric circuitry in the housing 1. A ground connector 11' or similar of the printed circuit board 43 is conveniently connected to the metallized portion of the ceramic-filled cavity 41''.

A sixth implantable medical device will next be described with reference to Figs. 8a-b. The device comprises as above an electrically conductive housing 1, in which electric circuitry 3, and radio frequency circuitry 5 are interconnected. Feed conductors 11, 13 are interconnected between the radio frequency circuitry 5 and edges 15, 17 of the slot, which in this example is a through hole 80 through the complete thickness of the housing 1. This eliminates the need of a cavity.

It shall be appreciated by the man skilled in the art that in an alternative version of this device, the through hole 80 is filled with dielectric material (not illustrated).

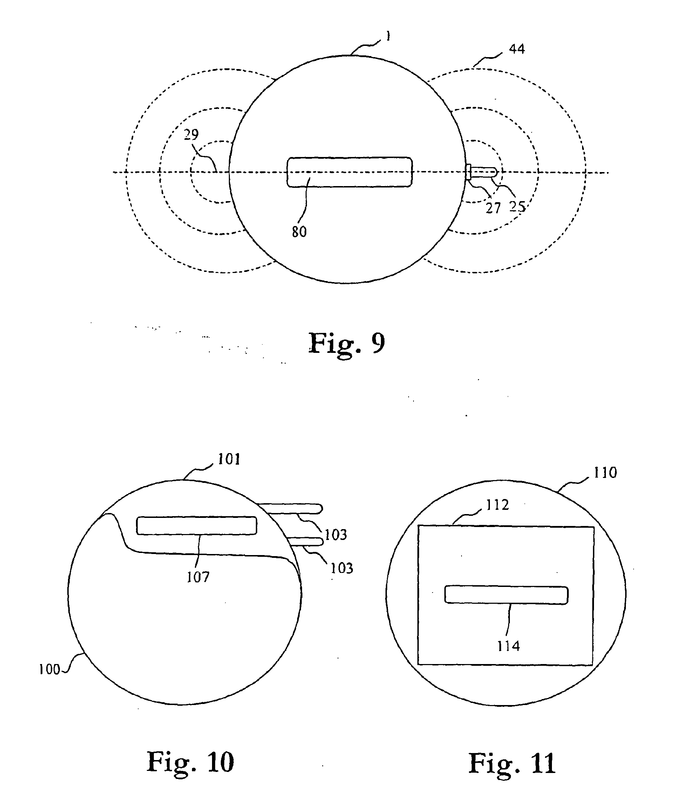

Fig. 9 illustrates a seventh implantable medical device wherein the device is provided with a therapy line 25 protruding from the housing 1 via a hermetic feed-through 27. The therapy line 25 is connected to the electric circuitry of the implantable medical device (not illustrated in Fig. 9 for sake of simplicity). The therapy line 25 protrudes from the housing 1 at a position along an extension line 29 of the slot 80. In other respects this device is identical with the device in Figs. 8a-b.

Generally, the therapy line 25 shall protrude from the housing 1 close to a voltage node of an electromagnetic field as obtained when the electrically conductive housing 1 operates as the antenna for the radio frequency signals. Such a voltage node can be found by calculating or measuring the electric field generated by the implantable medical device. In Fig. 9 equipotential surfaces of the electric field are denoted by 44.

Fig. 10 illustrates a preferred embodiment of the implantable medical device according to the invention. The hermetically sealed hollow housing 100 houses as in the devices above electric circuitry, radio frequency circuitry, an interconnection between them, and antenna feed conductors (not explicitly illustrated).

The housing 100 may be of an electrically conductive material or of a dielectric material such as e.g. a ceramic. Further, a header 101 is mounted to the housing 100, wherein the header is of a dielectric material and is advantageously a molded component, e.g. an injection molded part.

The header 101 supports two therapy lines 103, which are connected to the electric circuitry in the housing 100 via hermetic feed-throughs in the wall of the housing 100.

According to the present invention a portion of or the complete surface of the header 101 is covered by metal, preferably in a process known as metallization, and a slot antenna 107 is formed in the metallized surface, e.g. by means of patterning and etching. Alternatively, the patterned metallization with the slot 107 is formed by some kind of direct printing technique.

Generally, any of the different slots described in this description may be used in this embodiment: a slot which is closed in both ends thereof and which is optionally provided with a backing cavity, a slot which is closed in both ends thereof and which is formed as a through-hole to extend across the complete thickness of the header 101, and a notch antenna. In the two latter cases, the header 101 has naturally to be formed to include a through hole or a notch structure, before the surface is metallized.

The feed conductors are advantageously connected to the antenna in any of the manners as described in connection with the descriptions of the other devices herein. A hermetic feed-through for the feed conductors is provided in the wall of the housing 100.

If the housing 100 is of an electrically conductive material, the metallization of the header 101 is advantageously electrically connected to the housing 100.

Fig. 11 illustrates a ninth implantable medical device. The housing or cover, here denoted by 110, is of a material, which is not electrically conductive, such as e.g. a ceramic or a plastic. The electric circuitry and the radio frequency may be arranged separately in shielded and/or hermetically sealed compartments within the housing 110, or together in a single compartment. If they are arranged in separate compartments hermetic feed throughs are needed for the connection between them. If the circuitry is arranged in a hermetically sealed environment, the housing 110 itself does not necessarily have to be hermetically sealed. The housing 110 could for instance be molded.

The implantable medical device has a surface portion 112 made of an electrically conductive material, preferably a metal, wherein a slot 114 is provided in the surface portion 112 made of the electrically conductive material. A slot feeding is operatively interconnected between the radio frequency circuitry and the slot 114, and the surface portion 112 made of the electrically conductive material provided with the slot 114 is adapted to operate as a transmitting and/or receiving antenna for radio frequency signals transmitted and/or received by the radio frequency circuitry. The slot feeding may e.g. be similar to any of those illustrated in Figs. 5-7.

Preferably, the surface portion 112 made of the electrically conductive material is an outer surface portion of the housing 110, and thus of the medical device. The conductive surface portion 112 with the slot 114 can be formed as a patterned metallization of e.g. one side of the housing 110.

Alternatively, the conductive surface portion 112 is an internal surface within the housing 110. It may for instance be an outer surface of a metallic wall compartment within the housing 110, or a metallization of the surface of a dielectric wall compartment.

Still alternatively, the conductive surface portion 112 may be a piece of sheet metal, which is arranged within the housing 110.

In a further example, a large compartment or housing houses all components internal to the implantable medical device. It may be made of a metallic material or of a dielectric material, in which case a surface portion is metallized. The slot antenna is made in the wall of the compartment or housing, or in the metallized surface portion, and is connected as in any of the devices above. The complete compartment or housing is then covered by a dielectric material, preferably a thin layer of the dielectric material, to obtain a medical device having an outer surface suitable to be implanted in a human being, and optionally to hermetically seal the medical device. Only therapy lines, if any, have to be capable of being connectable to the interior of the medical device.

It shall be appreciated that the slots in devices as described above does not have to be formed along a straight line, but may have any suitable elongated shape.

The slot-based antenna is used in the present invention i.a. as is it can be formed on a metallic structure, the shape of which may be determined by special requirements, and which should not be changed by the presence of the slot.

It shall still further be appreciated that the implantable medical device may be provided with two or more slots with separate feeding. Thus, such an implantable medical device may be adapted for transmitting and/or receiving radio frequency waves in at least two different frequency bands, e.g. both around 400 MHz and around 2.4 GHz.

## Claims

1. An implantable medical device comprising:
- a housing (100);
- a header (101) adjacent said housing (100); and
- electric circuitry (3) for the operation of said implantable medical device and radio frequency circuitry (5) for transmitting and/or receiving radio frequency signals, said electric circuitry (3) and said radio frequency circuitry being interconnected (7) and arranged within said housing (100), wherein
- said implantable medical device has at least one surface portion (101) made of an electrically conductive material, and
- said at least one surface portion (101) made of the electrically conductive material is at least partly comprised of a portion of said header (101), the device further comprising
- at least one slot (107) that is provided in said at least one surface portion (101) made of the electrically conductive material in said header (101); and
- a slot feeding (11, 13; 11', 13') that is operatively interconnected between said radio frequency circuitry (5) and said at least one slot (107), wherein
- said at least one surface portion (101) made of said electrically conductive material provided with said at least one slot (107) is adapted to operate as a transmitting and/or receiving antenna for said radio frequency signals.

2. The device of claim 1 wherein said electrically conductive material is a metallic material.

3. The device of claim 1 or 2, comprising at least one therapy line (103), said at least one therapy line (103) being connected to said electric circuitry (3), and being arranged to protrude from said housing (100), and being supported by said header (101).

4. The device of claim 3, wherein
- said housing (100) is hermetically sealed; and
- said at least one therapy line (103) is provided with a hermetic feed-through (27) in said housing (100).

5. The device of any of claims 1-4, wherein
- said header (101) comprises a dielectric part and a metallization thereon; and
- said at least one surface portion (101) made of said electrically conductive material and at least partly comprised of said portion of said header (101) is said metallization.

6. The device of claim 5, wherein said slot (107) is provided in said metallization.

7. The device of claim 5 or 6, wherein said housing (100) is an electrically conductive housing (100), and said metallization is electrically connected to said electrically conductive housing.

8. The implantable medical device of any of claims 1-7, wherein said slot feeding (11, 13; 11', 13') is arranged across said at least one slot (10; 30; 50; 80).

9. The implantable medical device of any of claims 1-8, wherein said slot feeding (11, 13) includes center (13) and shield (11) conductors of a coaxial cable.

10. The implantable medical device of any of claims 1-9, wherein said at least one slot (30; 50; 80) is closed in both ends (33, 35) thereof.

11. The implantable medical device of claim 10, wherein said at least one slot (30; 50) is provided with an interior radiation shielding (40).

12. The implantable medical device of claim 10, wherein said at least one slot is a cavity-backed slot (50).

13. The implantable medical device of claim 10, wherein said at least one slot is a through hole (80) in said housing (1).

14. The implantable medical device of any of claims 1-9, wherein said at least one slot is a cavity-backed slot (50).

15. The implantable medical device of claim 14, wherein said slot feeding includes a coaxial feed-to-waveguide transition (11, 13, 41').

16. The implantable medical device of claim 14, wherein said slot feeding includes a wire feed-to-waveguide transition (11, 13, 41").

17. The implantable medical device of any of claims 1-16, wherein said at least one slot (10; 30; 50; 80) is arranged so that the extensions (21, 23) of said housing (1) on either side of said at least one slot (10 ; 30; 50; 80) in directions orthogonal to said at least one slot (10 ; 30; 50; 80) are of similar sizes.

18. The implantable medical device of any of claims 1-17, wherein said at least one slot is at least partially filled with a dielectric material (31), particularly a ceramic.

## Patentansprüche

1. Implantierbare medizinische Vorrichtung, umfassend:
- ein Gehäuse (100);
- einen Sockel (101) neben dem Gehäuse (100); und
- eine elektrische Schaltung (3) für den Betrieb der implantierbaren medizinischen Vorrichtung, und eine Hochfrequenzschaltung (5) zum Senden und/oder Empfangen von Hochfrequenzsignalen, wobei die elektrische Schaltung (3) und die Hochfrequenzschaltung zusammengeschaltet (7) und innerhalb des Gehäuses (100) angeordnet sind, wobei
- die implantierbare medizinische Vorrichtung mindestens einen Flächenabschnitt (101) aufweist, der aus einem elektrisch leitenden Material besteht, und
- wobei der mindestens eine Flächenabschnitt (101), der aus dem elektrisch leitenden Material hergestellt ist, mindestens teilweise aus einem Abschnitt des Sockels (101) besteht, wobei die Vorrichtung überdies umfasst
- mindestens einen Schlitz (107), der in dem mindestens einen Flächenabschnitt (101) vorgesehen ist, der aus dem elektrisch leitenden Material in dem Sockel (101) hergestellt ist; und
- eine Schlitzspeisung (11, 13; 11', 13'), die betriebsfähig zwischen der Hochfrequenzschaltung (5) und dem mindestens einen Schlitz (107) zwischengeschaltet ist, wobei
- der mindestens eine Flächenabschnitt (101), der aus dem elektrisch leitenden Material hergestellt ist, der mit dem mindestens einen Schlitz (107) versehen ist, angepasst ist, als Sende- und/oder Empfangsantenne für die Hochfrequenzsignale zu arbeiten.

2. Vorrichtung nach Anspruch 1, wobei das elektrisch leitende Material ein metallisches Material ist.

3. Vorrichtung nach Anspruch 1 oder 2, umfassend mindestens eine Behandlungsleitung (103), wobei die mindestens eine Behandlungsleitung (103) an die elektrische Schaltung (3) angeschlossen ist und angeordnet ist, von dem Gehäuse (100) hervorzustehen und von dem Sockel (101) gestützt zu werden.

4. Vorrichtung nach Anspruch 3, wobei
- das Gehäuse (100) hermetisch abgedichtet ist; und
- die mindestens eine Behandlungsleitung (103) mit einer hermetischen Durchführung (27) in dem Gehäuse (100) versehen ist.

5. Vorrichtung nach einem der Ansprüche 1 - 4, wobei
- der Sockel (101) einen dielektrischen Teil und eine Metallisierung darauf umfasst; und
- der mindestens ein Flächenabschnitt (101), der aus dem elektrisch leitenden Material hergestellt ist, und mindestens teilweise aus dem Abschnitt des Sockels (101) besteht, ist die Metallisierung.

6. Vorrichtung nach Anspruch 5, wobei der Schlitz (107) in der Metallisierung vorgesehen ist.

7. Vorrichtung nach Anspruch 5 oder 6, wobei das Gehäuse (100) ein elektrisch leitendes Gehäuse (100) ist, und die Metallisierung elektrisch an das elektrisch leitende Gehäuse angeschlossen ist.

8. Implantierbare medizinische Vorrichtung nach einem der Ansprüche 1 - 7, wobei die Schlitzspeisung (11, 13; 11', 13') über dem mindestens einen Schlitz (10; 30; 50; 80) angeordnet ist.

9. Implantierbare medizinische Vorrichtung nach einem der Ansprüche 1 - 8, wobei die Schlitzspeisung (11, 13) Mittel (13)- und Schirm (11)-Leiter eines Koaxialkabels umfasst.

10. Implantierbare medizinische Vorrichtung nach einem der Ansprüche 1 - 9, wobei der mindestens eine Schlitz (30; 50; 80) an beiden Enden (33, 35) davon geschlossen ist.

11. Implantierbare medizinische Vorrichtung nach Anspruch 10, wobei der mindestens eine Schlitz (30; 50) mit einer inneren Strahlenschirmung (40) versehen ist.

12. Implantierbare medizinische Vorrichtung nach Anspruch 10, wobei der mindestens eine Schlitz ein Hohlraumschlitz (50) ist.

13. Implantierbare medizinische Vorrichtung nach Anspruch 10, wobei der mindestens eine Schlitz ein Durchgangsloch (80) in dem Gehäuse (1) ist.

14. Implantierbare medizinische Vorrichtung nach einem der Ansprüche 1 - 9, wobei der mindestens eine Schlitz ein Hohlraumschlitz (50) ist.

15. Implantierbare medizinische Vorrichtung nach Anspruch 14, wobei die Schlitzspeisung einen Koaxialspeisung-Hohlleiter-Übergang (11, 13, 41') umfasst.

16. Implantierbare medizinische Vorrichtung nach Anspruch 14, wobei die Schlitzspeisung einen Drahtspeisung-Hohlleiter-Übergang (11, 13, 41") umfasst.

17. Implantierbare medizinische Vorrichtung nach einem der Ansprüche 1 - 16, wobei der mindestens eine Schlitz (10; 30; 50; 80) so angeordnet ist, dass die Verlängerungen (21, 23) des Gehäuses (1) an jeder Seite des mindestens einen Schlitzes (10; 30; 50; 80) in Richtungen orthogonal zu dem mindestens einen Schlitz (10; 30; 50; 80) von ähnlicher Größe sind.

18. Implantierbare medizinische Vorrichtung nach einem der Ansprüche 1 - 17, wobei der mindestens eine Schlitz mindestens teilweise mit einem dielektrischen Material (31), insbesondere einem keramischen Material, gefüllt ist.

## Revendications

1. Dispositif médical implantable comprenant :
- un boîtier (100) ;
- un socle (101) adjacent audit boîtier (100) ; et
- des circuits électriques (3) pour le fonctionnement dudit dispositif médical implantable et des circuits radiofréquence (5) pour émettre et/ou recevoir des signaux de radiofréquence, lesdits circuits électriques (3) et lesdits circuits radiofréquence étant interconnectés (7) et agencés à l'intérieur dudit boîtier (100), dans lequel
- ledit dispositif médical implantable présente au moins une portion de surface (101) fabriquée en un matériau électriquement conducteur, et
- ladite au moins une portion de surface (101) fabriquée en matériau électriquement conducteur comprend au moins en partie une portion dudit socle (101), le dispositif comprenant en outre
- au moins une fente (107) qui est fournie dans ladite au moins une portion de surface (101) fabriquée en matériau électriquement conducteur dans ledit socle (101) ; et
- une alimentation de fente (11, 13 ; 11', 13') qui est interconnectée de manière opérationnelle entre lesdits circuits radiofréquence (5) et ladite au moins une fente (107), dans lequel
- ladite au moins une portion de surface (101) fabriquée en ledit matériau électriquement conducteur pourvue de ladite au moins une fente (107) est adaptée pour fonctionner comme une antenne d'émission et/ou de réception pour lesdits signaux de radiofréquence.

2. Dispositif selon la revendication 1, dans lequel ledit matériau électriquement conducteur est un matériau métallique.

3. Dispositif selon la revendication 1 ou 2, comprenant au moins une ligne de thérapie (103), ladite au moins une ligne de thérapie (103) étant connectée auxdits circuits électriques (3), et étant agencée pour dépasser dudit boîtier (100), et étant supportée par ledit socle (101).

4. Dispositif selon la revendication 3, dans lequel
- ledit boîtier (100) est hermétiquement scellé ; et
- ladite au moins une ligne de thérapie (103) est pourvue d'une traversée hermétique (27) dans ledit boîtier (100).

5. Dispositif selon l'une quelconque des revendications 1-4, dans lequel
- ledit socle (101) comprend une partie diélectrique et une métallisation sur celle-ci ; et
- ladite au moins une portion de surface (101) fabriquée en ledit matériau électriquement conducteur et comprend au moins en partie ladite portion dudit socle (101) est ladite métallisation.

6. Dispositif selon la revendication 5, dans lequel ladite fente (107) est fournie dans ladite métallisation.

7. Dispositif selon la revendication 5 ou 6, dans lequel ledit boîtier (100) est un boîtier électriquement conducteur (100), et ladite métallisation est électriquement connectée audit boîtier électriquement conducteur.

8. Dispositif médical implantable selon l'une quelconque des revendications 1-7, dans lequel ladite alimentation de fente (11, 13 ; 11', 13') est agencée à travers ladite au moins une fente (10 ; 30 ; 50 ; 80).

9. Dispositif médical implantable selon l'une quelconque des revendications 1-8, dans lequel ladite alimentation de fente (11, 13) inclut des conducteurs central (13) et de blindage (11) d'un câble coaxial.

10. Dispositif médical implantable selon l'une quelconque des revendications 1-9, dans lequel ladite au moins une fente (30 ; 50 ; 80) est fermée à ses deux extrémités (33, 35).

11. Dispositif médical implantable selon la revendication 10, dans lequel ladite au moins une fente (30 ; 50) est pourvue d'un blindage intérieur contre les rayonnements (40).

12. Dispositif médical implantable selon la revendication 10, dans lequel ladite au moins une fente est une fente avec cavité (50).

13. Dispositif médical implantable selon la revendication 10, dans lequel ladite au moins une fente est un trou traversant (80) dans ledit boîtier (1).

14. Dispositif médical implantable selon l'une quelconque des revendications 1-9, dans lequel ladite au moins une fente est une fente avec cavité (50).

15. Dispositif médical implantable selon la revendication 14, dans lequel ladite alimentation de fente comprend une transition alimentation coaxiale - guide d'ondes (11, 13, 41').

16. Dispositif médical implantable selon la revendication 14, dans lequel ladite alimentation de fente comprend une transition alimentation par fil - guide d'ondes (11, 13, 41").

17. Dispositif médical implantable selon l'une quelconque des revendications 1-16, dans lequel ladite au moins une fente (10 ; 30 ; 50 ; 80) est agencée de sorte que les extensions (21, 23) dudit boîtier (1) de chaque côté de ladite au moins une fente (10 ; 30 ; 50 ; 80) dans des directions orthogonales à ladite au moins une fente (10 ; 30 ; 50 ; 80) sont de tailles similaires.

18. Dispositif médical implantable selon l'une quelconque des revendications 1-17, dans lequel ladite au moins une fente est au moins en partie remplie d'un matériau diélectrique (31), particulièrement de la céramique.
